Europäisches Patentamt

European Patent Office

Office européen des brevets

(11) Veröffentlichungsnummer : **0 275 301 B1**

(12) **EUROPÄISCHE PATENTSCHRIFT**

(19)

(45) Veröffentlichungstag der Patentschrift :
**15.01.92 Patentblatt 92/03**

(21) Anmeldenummer : **87905202.5**

(22) Anmeldetag : **29.07.87**

(86) Internationale Anmeldenummer :
**PCT/EP87/00411**

(87) Internationale Veröffentlichungsnummer :
**WO 88/01057 11.02.88 Gazette 88/04**

(51) Int. Cl.⁵ : **G01N 33/00, B60H 3/00**

(54) **VORRICHTUNG ZUR ERFASSUNG UNTERSCHIEDLICHER SCHADSTOFFGEHALTE VON GASSTRÖMEN.**

(30) Priorität : **02.08.86 DE 3626277**
**28.11.86 DE 3640734**

(43) Veröffentlichungstag der Anmeldung :
**27.07.88 Patentblatt 88/30**

(45) Bekanntmachung des Hinweises auf die
Patenterteilung :
**15.01.92 Patentblatt 92/03**

(84) Benannte Vertragsstaaten :
**AT BE CH DE FR GB IT LI LU NL SE**

(56) Entgegenhaltungen :
**EP-A- 0 042 287**
**DE-A- 3 304 324**
**DE-A- 3 423 848**
**US-A- 3 041 591**

(72) Erfinder : **IGELBÜSCHER, Heinrich**
**Marcq-en-Baroeul-Strasse 60**
**W-4390 Gladbeck (DE)**
Erfinder : **GRESCH, Heinrich**
**Franz-Lehàr-Strasse 25**
**W-4600 Dortmund-Wickede (DE)**
Erfinder : **DEWERT, Heribert**
**Bahnhofstrasse 23**
**W-4390 Gladbeck (DE)**
Erfinder : **BURGGRÄF, Peter**
**Melschedeweg 27**
**W-4630 Bochum 1 (DE)**
Erfinder : **HÖLTER, Heinz**
**Beisenstrasse 39-41**
**W-4390 Gladbeck (DE)**

(74) Vertreter : **Spalthoff, Adolf, Dipl.-Ing.**
**Pelmanstrasse 31 Postfach 34 02 20**
**W-4300 Essen 1 (DE)**

(73) Patentinhaber : **Hölter, Heinz, Dipl.-Ing.**
**Beisenstrasse 39-41**
**W-4390 Gladbeck (DE)**

## Beschreibung

Die Erfindung betrifft eine Vorrichtung zur Erfassung unterschiedlicher Schadstoffgehalte von Gasströmen, vorzugsweise für die Zufuhr zu Kraftfahrzeug- oder Arbeitsschutzkabinen, unter Verwendung eines schadstoffempfindlichen Halbleiter-Gassensors.

Es ist bekannt, schadstoffempfindliche Gassensoren zur Steuerung der Frischluft-/Umluftklappen und auch zusätzlich zur Ein- und Ausschaltung von Filtergeräten in Kraftfahrzeugkabinen und Arbeitsschutzkabinen zu verwenden. Weiterhin ist es bekannt, daß diese Halbleiter-Gassensoren, bedingt durch ihr Meßverfahren und ihre Herstellung, Toleranzen aufweisen, die für diesen Einsatzzweck unzulässig groß sind.

Es wurden deshalb zur Verbesserung der Empfindlichkeit und Stabilisierung dieser Sensoren zahlreiche Vorschläge gemacht, die jedoch nicht zum Erfolg führten. Beim Einsatz der schadstoffempfindlichen Gassensoren, z.B. im Kraftfahrzeug, ist die Querempfindlichkeit gegen unterschiedliche Fahrtgeschwindigkeiten, unterschiedliche Temperaturen, unterschiedliche Luftfeuchtigkeiten usw. noch immer unvertretbar groß.

Aus der DE-A 3 423 848 ist eine Vorrichtung zur Erfassung unterschiedlicher Schadstoffgehalte der Luft in einer Kraftfahrzeug- oder Arbeitsschutzkabine und der dieser zugeführten Außenluft unter Verwendung eines schadstoffempfindlichen Halbleiter-Gassensors bekannt, bei der zur Messung der Schadstoffkonzentration in der Außenluft ein Halbleiter-Gassensor vorgesehen ist, der der Außenluft ausgesetzt ist. Sofern dieser Sensor zu hohe Schadstoffbelastungen in der Außenluft erfaßt, wird die Zufuhr von Außenluft in die Kabine unterdrückt, wobei dann die Belüftung bzw. Klimatisierung der Kabine im Umluftbetrieb erfolgt.

Die US-A 30 41 591 zeigt eine Vorrichtung zur Erfassung der Schadstoffgehalte unterschiedlicher Gasströmungen oder -volumina, bei der der Sensor mittels einer Pumpe und einem entsprechend angeschlossenen Zuleitungssystem wahlweise einerseits mit dem Gas, dessen Schadstoffgehalt zu messen ist, und andererseits mit einem Vergleichsgas beaufschlagbar ist.

Aus der EP-A1-42 287 ist eine Vorrichtung zur Erfassung des Schadstoffgehalts einer in eine Fahrzeugkabine gerichteten Zuluftströmung beschrieben. Hierbei ist der Schadstoffsensor innerhalb des Zufuhrkanals angeordnet. Vor dem Schadstoffsensor sitzt eine umschaltbare Luftklappe, mittels der der Zufuhrkanal sowohl an die Außenluft als auch an die Innenluft angeschlossen werden kann. Der Schadstoffsensor mißt jeweils die innerhalb des Zufuhrkanals herrschende Schadstoffkonzentration. Eine Messung der Schadstoffkonzentration der Innenluft bzw. der Schadstoffkonzentration der Außenluft unabhängig von der Stellung der vor dem Schadstoffsensor im Zufuhrkanal angeordneten Luftklappe ist nicht vorgesehen.

Ausgehend von dem vorstehend geschilderten Stand der Technik liegt der Erfindung die Aufgabe zugrunde, eine Vorrichtung zur Erfassung unterschiedlicher Schadstoffgehalte der Luft in einer Kraftfahrzeug- oder Arbeitsschutzkabine und der dieser zugeführten Außenluft zu schaffen, bei der die Zufuhr von Außenluft in die Kabine in Abhängigkeit vom Schadstoffgehalt sowohl der Außen- als auch der Kabinenluft geregelt wird, wobei die Vorrichtung einen einfachen Aufbau aufweisen soll.

Diese Aufgabe wird erfindungsgemäß dadurch gelöst, daß dem Gassensor eine Umschaltvorrichtung zur wechselweisen Beaufschlagung des Gassensors mit Innen- und Außenluft zugeordnet ist und daß der Gassensor und die Umschalteinrichtung auf der Frischluft-/Umluftklappe angeordnet sind. Durch die erfindungsgemäßen Maßnahmen ist der Gassensor an der Trennstelle zwischen der Außen- und der Kabinenluft angebracht. Durch Umschalten, das im Bereich von wenigen Sekunden erfolgt, ist praktisch ständig ein Vergleich zwischen der Qualität der Innen- und der Außenluft möglich.

Vorteilhaft kann das Ausgangssignal des Gassensors zur Steuerung der Frischluft-/Umluftklappe dienen.

Es ist bekannt, daß Halbleiter-Sensoren, insbesondere auf Zinndioxid-Basis, sehr stark querempfindlich sind gegenüber Temperatur- und Feuchteschwankungen. Teilweise sind die Schwankungen derart groß, daß bis zu 70 % Meßwertverschiebungen auftreten können, und zwar durch unterschiedliche Feuchte. Ähnlich groß ist der Einfluß bei Temperaturschwankungen. Bekannt ist es, über Temperaturfühler eine Temperaturkompensation herbeizuführen. Die Feuchte läßt sich technisch nur als relative Feuchte messen. Dieser Meßwert führt jedoch zu ganz unterschiedlichen absoluten Feuchten bei unterschiedlicher Lufttemperatur, so daß das Maß der relativen Feuchte nicht unmittelbar zur Korrektur des Feuchteneinflusses herangezogen werden kann. Daher wird die Korrektur sehr aufwendig, da eine solche im allgemeinen nur über einen Rechner gelöst werden kann.

In einer vorteilhaften Ausführungsform der Erfindung sind zur Korrektur von Temperatur- und Feuchteschwankungen des Meßgases ein Temperaturfühler, ein Feuchtesensor und ein Mikroprozessor vorhanden; mittels des Mikroprozessors wird der Meßwert des Gassensors mit einem Korrekturfaktor korrigiert, der aus einem Mittelwert aus Temperatur und Feuchtigkeit bestimmt wird.

Die Temperatur- und Feuchtigkeitswerte sind in einer zweidimensionalen Matrix festgelegt, so daß in einem Mikroprozessor von nur 4 K Kapazität ein Feld mit 200 Werten abgespeichert werden kann, was völlig ausreicht, um eine genügend genaue Kompensation vornehmen zu können.

Ausführungsbeispiele der Erfindung sind an Hand der Zeichnung näher erläutert, und zwar zeigt:

Figur 1 in schematischer Darstellung ein Kraftfahrzeug mit einer Vorrichtung zur Erfassung unterschiedlicher Schadstoffgehalte von Gasströmen und

Figur 2 eine Schaltungsanordnung zur Korrektur von Temperatur- und Feuchteschwankungen eines Gasstromes.

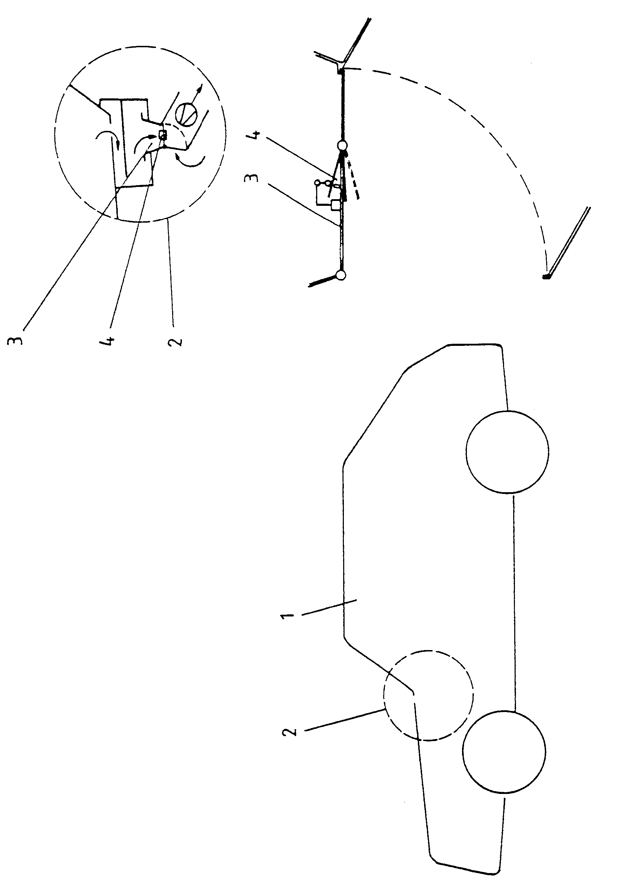

In Figur 1 ist mit 1 das Kraftfahrzeug und mit 2 der Bereich der Fahrzeugluftversorgung bezeichnet. Der schadstoffempfindliche Gassensor 4 ist an der Trennstelle zwischen Außenluft und Kabinenluft angebracht, wobei dieser mit einer Umschalteinrichtung versehen ist. Der Gassensor 4 mit seiner Umschalteinrichtung ist auf der Umluftklappe 3 angeordnet.

Durch diese Ausgestaltung ist gewährleistet, daß der bei einer Anbringung an einem stationären Meßort benötigte Sensor zur Erfassung der absoluten Größe der Schadstoffverunreinigung nicht mehr unter enormem Prüf- und Einstellaufwand angebracht werden muß, sondern es ist jetzt die Möglichkeit gegeben, einfache Sensoren zu verwenden, die den Unterschied zwischen besserer oder schlechterer Luft sicher bewerten können und letztendlich das Ausgangssignal zur Steuerung der Frischluft-/Umluftklappe geben können.

Der Grundgedanke dieser Ausführung besteht somit darin, die Qualität zweier Luftströme mittels eines schadstoffempfindlichen Gassensors unter Zurhilfenahme einer mechanischen Umschaltvorrichtung laufend vergleichend zu bewerten.

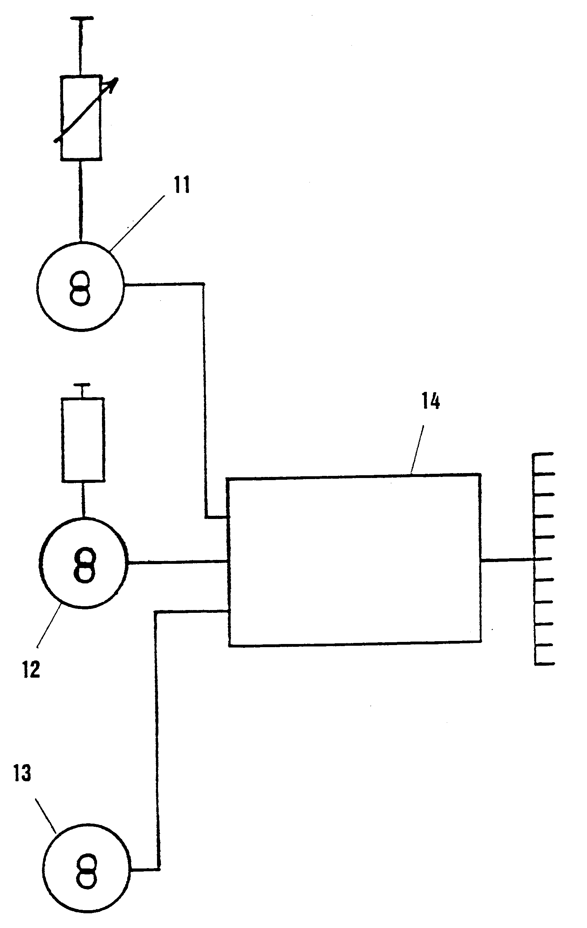

Bei der Ausführung nach Figur 2 ist mit 11 der Gassensor, mit 12 der Temperaturfühler und mit 13 der Feuchtesensor bezeichnet. Die drei Sensoren 11, 12, 13 geben gemeinsam ihre Werte in den Mikroprozessor 14 ab.

Der Mikroprozessor 14 sucht für die Temperatur- und Feuchtewerte den jeweils entsprechenden Korrekturwert heraus, mit dem der Gassensorwert korrigiert wird.

Dadurch wird ein temperatur- und feuchteunabhängiges Ausgangssignal erhalten.

**Patentansprüche**

1. Vorrichtung zur Erfassung unterschiedlicher Schadstoffgehalte der Luft in einer Kraftfahrzeug- oder Arbeitsschutzkabine und der dieser zugeführten Außenluft unter Verwendung eines schadstoffempfindlichen Halbleiter-Gassensors (4), dadurch gekennzeichnet, daß dem Gassensor (4) eine Umschaltvorrichtung zur wechselweisen Beaufschlagung des Gassensors mit Innen- und Außenluft zugeordnet ist und daß der Gassensor und die Umschalteinrichtung auf der Frischluft-/Umluftklappe (3) angeordnet sind.

2. Vorrichtung nach Anspruch 1, dadurch gekennzeichnet, daß das Ausgangssignal des Gassensors zur Steuerung der Frischluft/Umluftklappe dient.

3. Vorrichtung nach Anspruch 1, dadurch gekennzeichnet, daß zur Korrektur von Temperatur- und Feuchtschwankungen des Meßgases ein Temperaturfühler (12), ein Feuchtesensor (13) und ein Mikroprozessor (14) vorhanden sind und daß mittels des Mikroprozessors der Meßwert des Gassensors (11) mit einem Korrekturfaktor korrigierbar ist, welcher aus einem Mittelwert aus Temperatur und Feuchtigkeit bestimmt wird.

**Claims**

1. Device for detecting different contents of pollutants in the air of a motor vehicle or protective working cabin and of the outer air delivered thereto by using a pollutant-sensitive semiconductor gas sensor (4), characterised in that the gas sensor (4) is associated with a switching device for alternately acting upon the gas sensor with inner and outer air, and in that the gas sensor and the switching device are disposed on the fresh air-/recirculated air flap (3).

2. Device according to claim 1, characterised in that the output signal of the gas sensor serves to control the fresh air-/recirculated air flap.

3. Device according to claim 1, characterised in that a temperature sensor (12), a moisture sensor (13) and a microprocessor (14) are provided in order to correct the temperature and moisture fluctuations of the measuring gas; and in that by means of the microprocessor the measuring value of the gas sensor (11) can be corrected with a correcting factor which is calculated from an average value of temperature and moisture.

**Revendications**

1. Dispositif pour la détection des teneurs différentes en substances toxiques de l'air dans un habitacle de véhicule automobile ou dans une cabine de protection et de l'air extérieur envoyé dans celle-ci, utilisant un détecteur de gaz à semi-conducteur (4) réagissant aux substances toxiques, caractérisé en ce qu'un dispositif de commutation pour alimenter alternativement le détecteur de gaz avec de l'air intérieur et de l'air extérieur est associé au détecteur de gaz (4) et en ce que le détecteur de gaz et le dispositif de commutation sont montés sur le volet d'air frais/air ambiant (3).

2. Dispositif selon la revendication 1, caractérisé en ce que le signal de sortie du détecteur de gaz est utilisé pour la commande du volet d'air frais/air ambiant.

3. Dispositif selon la revendication 1, caractérisé en ce qu'il est prévu, pour corriger les variations de température et d'humidité du gaz mesuré, un capteur de température (12), un capteur d'humidité (13) et un microprocesseur (14) et en ce que la valeur mesurée par le détecteur de gaz (11) peut être corrigée par le microprocesseur à l'aide d'un facteur de correction qui est déterminé à partir de la température et de l'humidité.